# EUROPEAN PATENT SPECIFICATION

(11) **EP 0 547 708 B1**
(45) Date of publication and mention of the grant of the patent: **26.02.2003**
(21) Application number: 92203917.7
(22) Date of filing: 15.12.1992
(51) Int. Cl.: C07D 275/06, C07D 417/12, C07D 417/14, C07D 471/04, A61K 31/425

(54) **Saccharin derivative proteolytic enzyme inhibitors**
Saccharinderivate als proteolytische Enzyminhibitoren
Inhibiteurs d'enzymes protéolytiques dérivés de la saccharine

(30) Priority: 19.12.1991 US 810265
(43) Date of publication of application: 23.06.1993
(73) Proprietor: SANOFI-SYNTHELABO, 75013 Paris (FR)
(72) Inventor: Hlasta, Dennis John, c/o STERLING WINTHROP INC., New York, New York 10016 (US); Ackerman, James Howard, c/o STERLING WINTHROP INC., New York, New York 10016 (US); Mura, Albert Joseph, c/o EASTMAN KODAK COMPANY, Rochester, New York 14650-2201 (US)
(74) Representative: Bernasconi, Jean Raymond

(56) References cited:
- EP-A- 0 253 092
- EP-A- 0 483 928
- WO-A-90/13549
- FR-A- 2 321 288
- US-A- 4 276 298

## Description

The invention relates to saccharin derivatives which inhibit the enzymatic activity of proteolytic enzymes, to processes for preparation thereof, to method of use thereof in the treatment of degenerative diseases and to pharmaceutical compositions thereof.

Inhibitors of proteolytic enzymes are useful in treatment of which proteolysis is a substantive element. Serine proteases are the most widely distributed class of proteolytic enzymes. Some serine proteases are characterized as chymotrypsin-like or elastase-like based upon their substrate specificity. Chymotrypsin and chymotrypsin-like enzymes normally cleave a peptide bond in a protein at a site at which the amino acid on the carbonyl side is Trp, Tyr, Phe, Met, Leu or other amino acid which contains an aromatic or a large alkyl side chain. Elastase and elastase-like enzymes normally cleave a peptide bond at a site at which the amino acid residue on the carbonyl side of the bond is Ala, Val, Ser, Leu or other small amino acid. Both chymotrypsin-like and elastase-like enzymes are found in leukocytes, mast cells and pancreatic juice in higher organisms, and are secreted by many types of bacteria, yeast and parasites.

WO-A-9013549 describes a series of 2-substituted saccharin derivatives useful as proteolytic enzyme inhibitors. Further saccharin derivatives are known from US-A-4276298 EP-A- 0483928, FR-A-2321288, EP-A-0253092 and EP-A-0542372

According to the present invention there is provided a compound having the structural formula 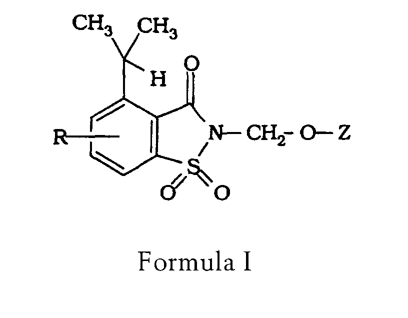 wherein
R is hydrogen or methoxy ; and
Z is selected from the following radicals : 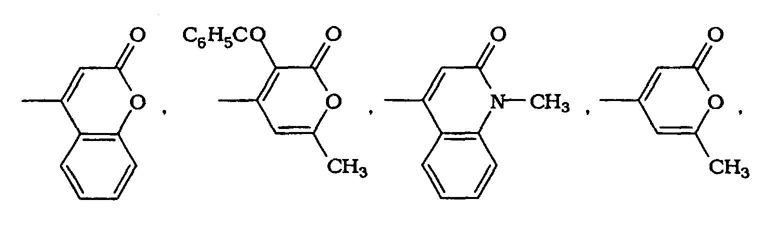 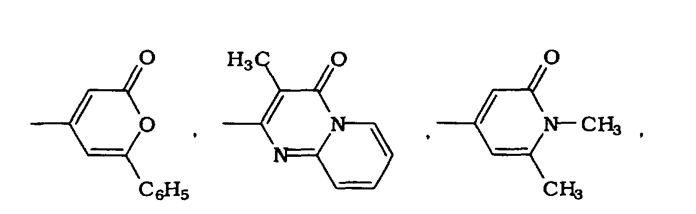 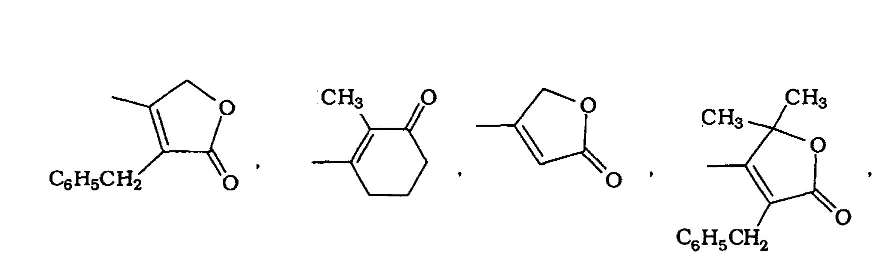 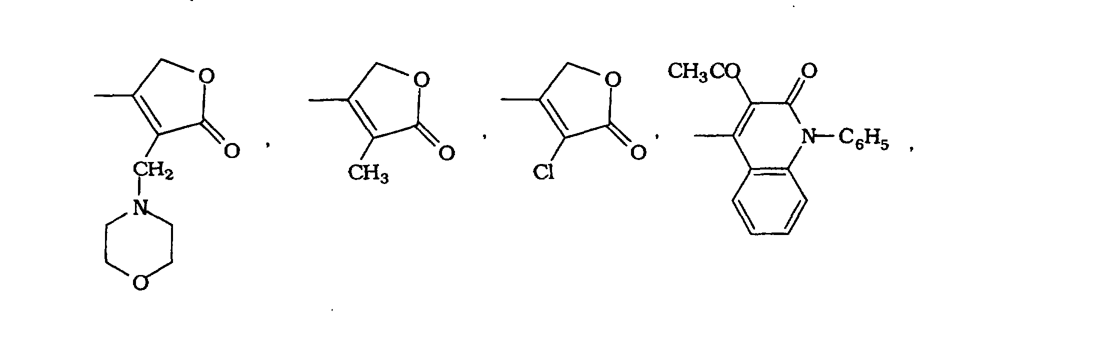 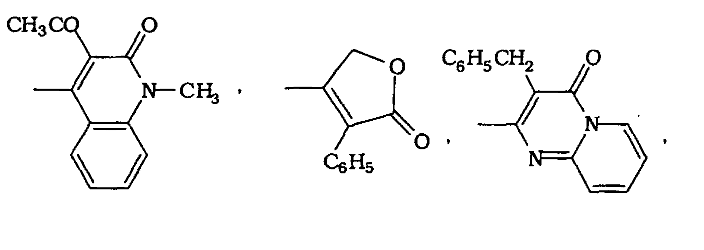 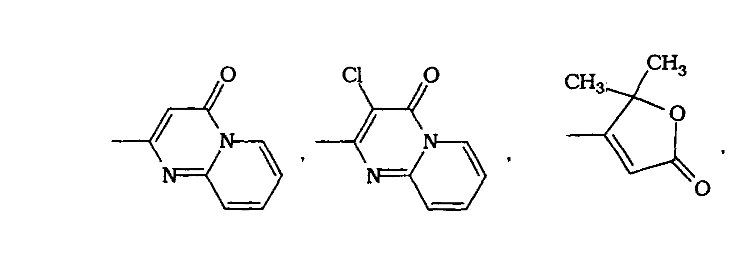 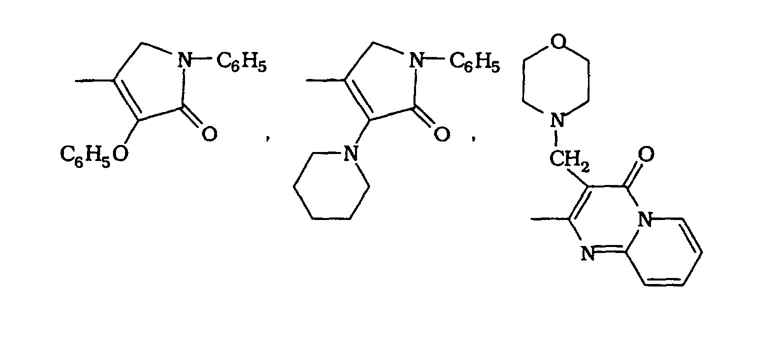 or a pharmaceutically acceptable acid addition salt thereof if the compound has a basic functional group or a pharmaceutically acceptable base addition salt thereof if the compound has an acidic functional group.

Preferred compounds of formula I are those in which R is hydrogen or 6-methoxy.

The compounds of Formula I inhibit the enzymatic activity of proteolytic enzymes and are useful in the treatment of degenerative diseases.

The invention further provides a process for preparing a compound of Formula I which comprises condensing the corresponding compound having the structural formula: 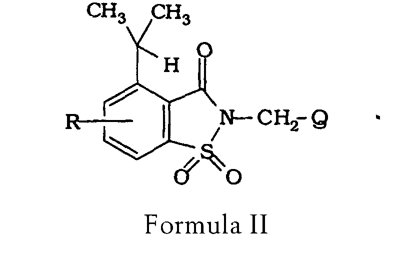 in which is as defined above, wherein Q is chloro or bromo with the corresponding compound having the structural formula (III):

H-O-Z Formula III

wherein Z is as defined above, in the presence of a base or with a corresponding basic salt of the compound of formula III.

In another aspect the invention provides the use of a compound of Formula I for preparation of a medicament for the treatment of a degenerative disease in a patient in need of such treatment.

In yet another aspect there is provided a pharmaceutical composition for the treatment of degenerative disease which comprises a proteolytic enzyme inhibiting concentration of a compound of Formula I in a pharmaceutical carrier.

In carrying out the preparation of a compound of Formula I from a corresponding compound of Formula II and the corresponding compound of Formula III in the presence of a base, the base can be any base which is not itself a reactant under the reaction conditions and is preferably an alkali metal carbonate, an alkali metal alkoxide, a tri-lower-alkylamine, a thallous lower-alkoxide, 1,8-diazabicyclo[5.4.0]undec-7-ene or 7-methyl-1,5,7-triazabicyclo[4.4.0]dec-5-ene. Under the reaction conditions the base may form the basic salt of the compound of Formula III, which then reacts with the compound of Formula II. The basic salt of the compound of Formula III can also be formed separately and then condensed with the compound of Formula II and is preferably an alkali metal, especially cesium, or thallous salt thereof. The condensation is carried out in an organic solvent or mixture of organic solvents inert under the reaction conditions, for example acetone, methylethylketone, acetonitrile, tetrahydrofuran, diethyl ether, dimethylformamide, N-methylpyrrolidone, dichloromethane, xylene, toluene or a lower-alkanol or mixture thereof, at a temperature in the range from ambient temperature to the boiling temperature of the solvent or solvent mixture.

The compounds of Formulas II and III are known or are made by known methods or by methods described below.

A compound of Formula II can be prepared by diazotizing the corresponding lower-alkyl 2-aminobenzoate ester, chlorosulfonylating the resulting lower-alkyl benzoate ester 2-diazonium salt with sulfur dioxide and cuprous chloride; and cyclizing the resulting lower-alkyl 2-chlorosulfonylbenzoate ester with ammonia to form the corresponding compound having the structural formula: 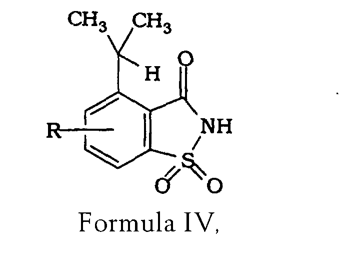 hydroxymethylation of which with formaldehyde affords the corresponding 2-hydroxymethyl-saccharin, displacement of whose hydroxyl with chloride or bromide using, for example thionyl chloride, thionyl bromide, phosphorus trichloride or phosphorus tribromide affords the corresponding compound of Formula II.

A compound of Fomula II wherein Q is chloro can also be prepared in one step from the corresponding compound of Formula IV by chloromethylation with paraformaldehyde and chlorotrimethylsilane in the presence of a Lewis acid, for example stannic chloride.

A compound of Formula IV can be prepared by lithiating the corresponding 4-methyl-saccharin with two molar equivalents of a lower-alkyl lithium in an inert solvent, for example tetrahydrofuran, and alkylating the resulting 4-lithiomethyl-5, 6 or 7-R₂-saccharin with the appropriate alkyl halide. Both reactions are carried out at a temperature in the range from -80°C. to - 50°C.

A compound of Formula IV can also be prepared by introducing. The 4-isopropyl group earlier in the synthesis. Conjugate addition of the appropriate alkyl-cuprate to 2-cyclo-hexenone and methoxycarbonylation of the resulting copper enolate with methyl cyanoformate by the method of Winkler et al. (Tetrahedron Letters, p. 1051, 1987; Journal of Organic Chemistry, vol. 54, p.4491 1989) gives the corresponding 2-methoxycarbonyl-3 isopropyl-cyclohexanone, enol etherification of which with benzylthiol and acidic clay gives a mixture of the corresponding 6-2-benzylthio-1-cyclohexenecarboxylic acid methyl ester and 6-2-benzylthio-3-cyclohexenecarboxylic acid methyl ester, aromatization of which with dichlorodicyanobenzoquinone gives the corresponding 2-6-benzylthiobenzoic acid methyl ester, oxidation-chlorination-debenzylation of which with chlorine in aqueous acetic acid gives 2-6-chlorosulfonylbenzoic acid methyl ester, cyclization of which with ammonia gives the corresponding 4-isopropyl-saccharin of Formula IV' .

The pharmaceutically acceptable acid or base addition salt can be any pharmaceutically acceptable acid or base addition salt but preferably has a common anion, for example the hydrochloride salt, or common cation, for example the sodium or potassium salt, respectively. If the salt having a common anion or cation is unacceptable because it is not crystalline or insufficiently soluble or hygroscopic, a salt having a less common anion, for example the methane-sulfonate, or less common cation, for example the diethylammonium salt, can be used respectively. In any event for use in a mammal the acid or base addition salt must be nontoxic and must not interfere with the elastase inhibitory effect of the free base or acid form respectively of the compound of Formula I.

In the preparations and examples described below the structures of the products are inferred from known structures of starting materials and expected courses of preparative reactions. Purification or purity and structural confirmation of starting materials and products were carried out or measured by melting temperature range (m.r.), optical rotation, elemental analysis, infrared spectral analysis, ultraviolet spectral analysis, mass spectral analysis, nuclear magnetic resonance spectral analysis, gas chromatography, column chromatography, high pressure liquid chromatography, medium pressure liquid chromatography and/or thin layer chromatography.

The invention will now be described with reference to the following examples.

### Example 1 Preparation of Starting Materials

### (a) Preparation of 2-Chloromethyl-4-isopropylsaccharin

n-Butyllithium (2.5 M, 100 ml) was added with stirring under nitrogen at 0-5°C. during ten minutes to a solution of 2-isopropylbromobenzene in anhydrous ether (500 ml). The mixture was allowed to warm to room temperature, stirred at room temperature for six hours, and cooled to -60°C. A solution of diethylcarbamyl chloride (34 g) in anhydrous ether (50 ml) was added during 20 minutes while maintaining the temperature below -50°C. The temperature was allowed to rise to room temperature during one hour. Water (100 ml) was added. The ether layer was washed with saturated aqueous sodium chloride (200 ml), dried over magnesium sulfate and stripped of ether. Distillation of the residue (80-90°C./0.1 mm Hg) gave 2-isopropyl-N N-diethylbenzamide (44 g, 80% yield).

To a solution of N,N,N',N'-tetramethylethylenediamine (25.5 g) in anhydrous ether (600 ml) was added s-butyllithium (1.3 M, 170 ml) and the mixture was cooled to -70°C. under nitrogen. A solution of 2-isopropyl-N,N-diethylbenzamide (44 g) in anhydrous ether (300 ml) was added dropwise over 20 minutes. The temperature was maintained at or below -60°C. during the addition. After the addition the mixture was stirred at -70°C. for 30 minutes, allowed to warm to -50°C. during 30 minutes, held at -50°C.for 10 minutes, then cooled back to -70°C. By cannulation tube a solution of sulfur dioxide (50 g) in anhydrous ether (50 ml) precooled to -60°C. was added under positive nitrogen pressure over a 10-minute period. The temperature of the reaction mixture during the addition was maintained below -50°C. A white powdery precipitate of aryllithium sulphinate separated out almost immediately. The temperature was allowed to rise to room temperature during one hour. Sulfuryl chloride (54 g) was added dropwise with continued stirring during 15 minutes. After further stirring for 30 minutes at 0-5°C. a white precipitate was filtered off and washed with anhydrous ether (2 l). Removal of the solvent under vacuum gave a faint yellow oil, which was dissolved in tetrahydrofuran (150 ml). The solution was cooled to 0°C. and concentrated aqueous ammonia (28%, 60 ml) was added in portions over 15 minutes. The temperature was kept at 10°C. or below throughout the addition. After stirring for 15 minutes at ambient temperature the tetrahydrofuran and excess ammonia were removed and the residue was acidified with hydrochloric acid (2N) to pH 1. The resulting white solid was collected, washed with water (200 ml) and hexane (200 ml) and dried affording 2-aminosulfonyl-6-isopropyl N-N-diethylbenzamide (54 g, 90% yield).

A solution of 2-aminosulfonyl-6-isopropyl-N,N-diethylbenzamide (60 g) in acetic acid (400 ml) was refluxed for 24 hours, then cooled to room temperature. The solvent was removed under vacuum. The oily residue was dissolved in water (500 ml) and the pH was adjusted to 1 with 2N hydrochloric acid. The crude product was collected by filtration, washed with water (300 ml), dried at 60°C. under vacuum for 18 hours and recrystallized from ether-hexane to give 4-isopropylsaccharin (40 g, 90% yield, m.p. 177°C.).(IV; R₁=iPr, R₂=H)

A mixture of 4-isopropylsaccharin (37.9 g), phenyl chloromethyl sulfide (33.3 g), tetrabutylammonium bromide (5.4 g) and toluene (200 ml) was heated under reflux for 24 hours, then stripped of volatiles. Column chromatography of the residue on silica gel (485 g) and elution first with hexane, then hexane-dichloromethane (1:1), then dichoromethane gave in the hexane-dichloromethane eluate 2-phenylthiomethyl-4-isopropylsaccharin as a pale yellow oil (53.5 g, 92% yield).

2 2-Phenylthiomethyl-4-isopropylsaccharin (53.5 g), sulfuryl chloride (40 ml, 67.2 g) and dichloromethane (250 ml) were mixed with stirring at room temperature. The mixture underwent a slightly exothermic reaction and was allowed to stand for 17 hours at room temperature, then stripped of volatiles. The residue crystallized with hexane in three crops (33.65 g, m.r. 101-102°C.; 3.45 g, m.r. 100-101°C.; 0.45 g, m.r. 99-100°C.; total, 38.55 g, 91% yield). The three crops were combined and recrystallized from isopropyl alcohol (30 ml)-cyclohexane (270 ml) affording 2-chloromethyl-4-isopropylsaccharin in two crops (33.5 g, m.r. 101-102.5°C.; 2.65 g, m.r. 100-101°C.).

### (b) Preparation of 2-Chloromethyl-4-isopropyl-6-methoxysaccharin

To a solution of N,N,N',N'-tetramethylethylenediamine (300 ml) in anhydrous ether (41) was added s-butyllithium (1.3 M, 4 l) and the mixture was cooled to -70°C. under nitrogen. A solution of 2-isopropyl-4-methoxy-N,N-diethylbenzamide (454.2 g) in anhydrous ether (300 ml) was added dropwise over 30 minutes. The temperature was maintained at or below -60°C. during the addition. After the addition the mixture was stirred at -70°C. for one hour, allowed to warm to -50°C., held at -50°C.for 30 minutes, then cooled back to -70°C. By cannulation tube a solution of sulfur dioxide (200 g) in anhydrous ether (200 ml) precooled to -40°C. was added under positive nitrogen pressure over a 20-minute period. The temperature of the reaction mixture during the addition was maintained below -40°C. A white powdery precipitate of aryllithium sulphinate separated out almost immediately. After the addition the cooling bath was removed and the mixture was stirred at ambient temperature for two hours, then cooled to -5°C. With continued stirring sulfuryl chloride (190 ml) was added dropwise over a 15-minute period while maintaining the temperature below 10°C. After further stirring for 30 minutes at 0-5°C. a white precipitate was filtered off and washed with anhydrous ether (2 l). Removal of the solvent at atmospheric pressure gave a dark oil, which was dissolved in tetrahydrofuran (1.4 l). The solution was cooled to -10°C. and concentrated aqueous ammonia (28%, 540 ml) was added in portions over 15 minutes. The temperature was kept at 15°C. or below throughout the addition. After stirring for 15 minutes at ambient temperature the tetrahydrofuran and excess ammonia were removed under vacuum to give a dark oil, which was diluted with water (6.0 l) and acidified with hydrochloric acid (3N) to pH 1. The resulting light yellow solid was collected by filtration, washed with water (800 ml), dried at 60°C. under vacuum for 18 hours and recrystallized from ethyl acetate-hexane (800 ml-3 l) to give 2-aminosulfonyl-6-isopropyl-4-methoxy-N,N-diethylbenzamide (429 g, 72% yield, m r. 122-125°C.).

A solution of 2-aminosulfonyl-6-isopropyl-4-methoxy-N,N-diethylbenzamide (429.6 g) in acetic acid (1.5 l) was refluxed for 20 hours, then cooled to room temperature. The solvent was removed under vacuum. The oily residue was dissolved in water (6 l) and the pH was adjusted to 1 with hydrochloric acid (6N). The crude product was collected by filtration, washed with water (2 l), dried at 60°C. under vacuum for 18 hours and recrystallized from ethyl acetate-hexane to give 4-isopropyl-6-methoxysaccharin (303g, 91% yield, m.p. 188°C.).

To a suspension of paraformaldehyde (24 g) and chlorotrimethylsilane (86.4 g) in 1,2-dichloroethane (200 ml) was added dry tin(IV) chloride (0.8 ml) and the resulting solution was stirred on a steam bath for one hour. 4-Isopropyl-6-methoxysaccharin (51.4 g) was added to the clear solution and the mixture was refluxed for 18 hours, cooled to room temperature and poured into water. The organic layer was separated, washed with aqueous sodium hydroxide solution (2N, 50 ml), dried over magnesium sulfate and concentrated under vacuum. The residue was crystallized from ethyl acetate-hexane to give 2-chloromethyl-4-isopropyl-6-methoxysaccharin (57 g, 87% yield, m.p. 151°C.).

### Preparation of Compounds of Formula I

### Example 1A

### 2-[(2,5-dihydro-5-oxo-3-furanyl)oxymethyl]-4-isopropylsaccharin

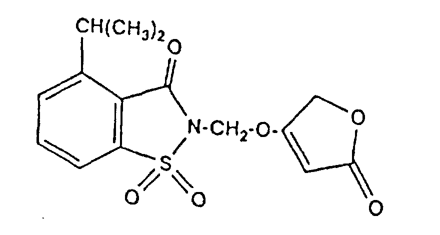

A solution of tetronic acid (0.22 g) in dimethylformamide (5 ml) was added to a suspension of sodium hydride (60% dispersion in mineral oil, 0.10 g) in dimethylformamide (4 ml) under nitrogen with stirring and ice-bath cooling. The ice bath was removed, stirring was continued for 15 minutes, a solution of 2-chloromethyl-4-isopropylsaccharin (0.547 g) in dimethylformamide (10 ml) was added dropwise, stirring was continued for two and one-half days, and the mixture was poured into water. The resulting mixture was extracted with ethyl acetate. The ethyl acetate layer was dried over magnesium sulfate and stripped of ethyl acetate. Recrystallization of the resulting colorless solid (0.60 g) from ethyl acetate gave the title compound (0.34 g, 51% yield, m.r. 174-175°C.).

### Examples 1B-1R

By methods similar to that of Example 1A above the compounds identified in Table I below were prepared from 2-chloromethyl-4-isopropylsaccharin or 2-chloromethyl-4-isopropyl-6-methoxysaccharin and the corresponding compound of Formula III (H-O-Z).

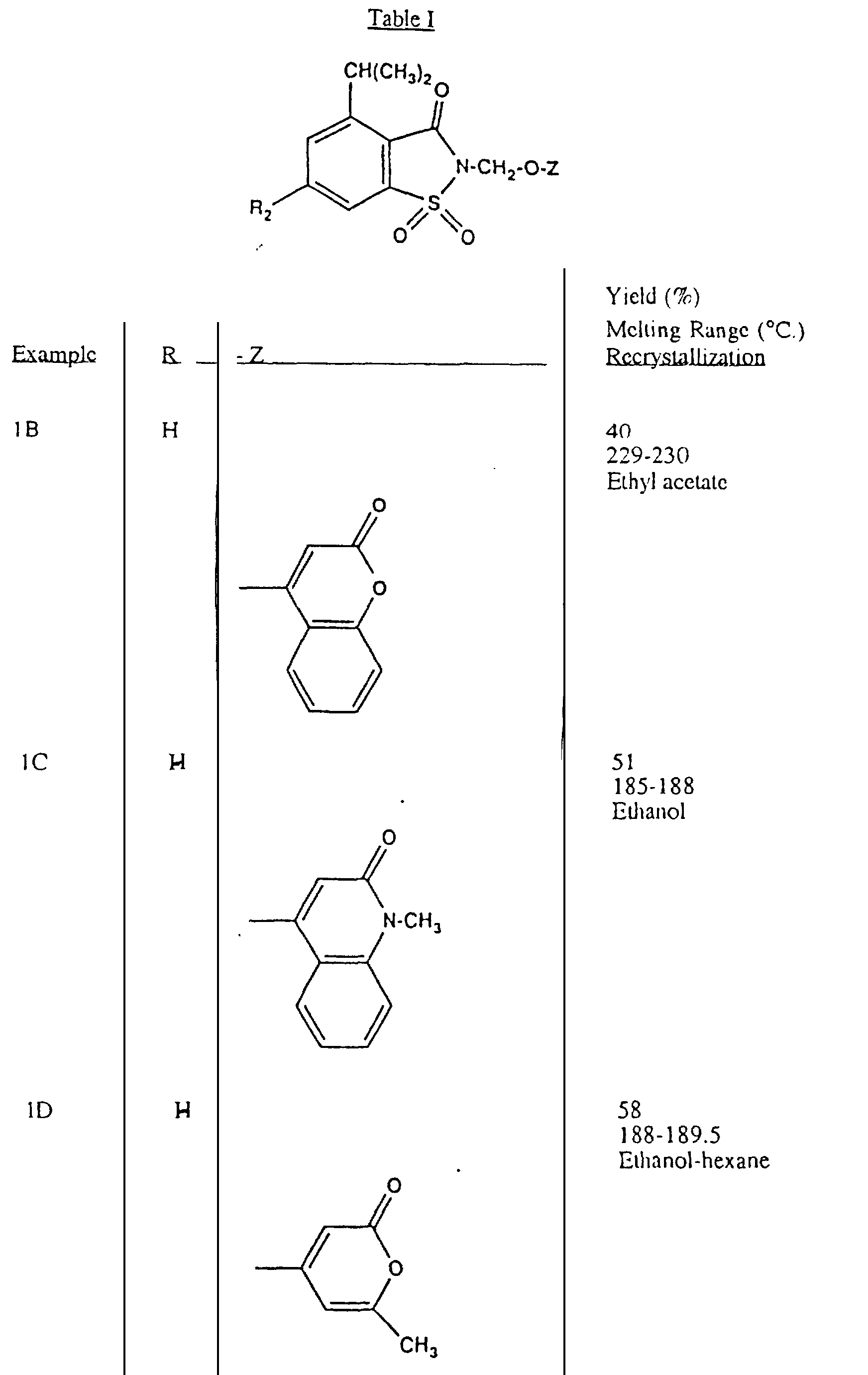

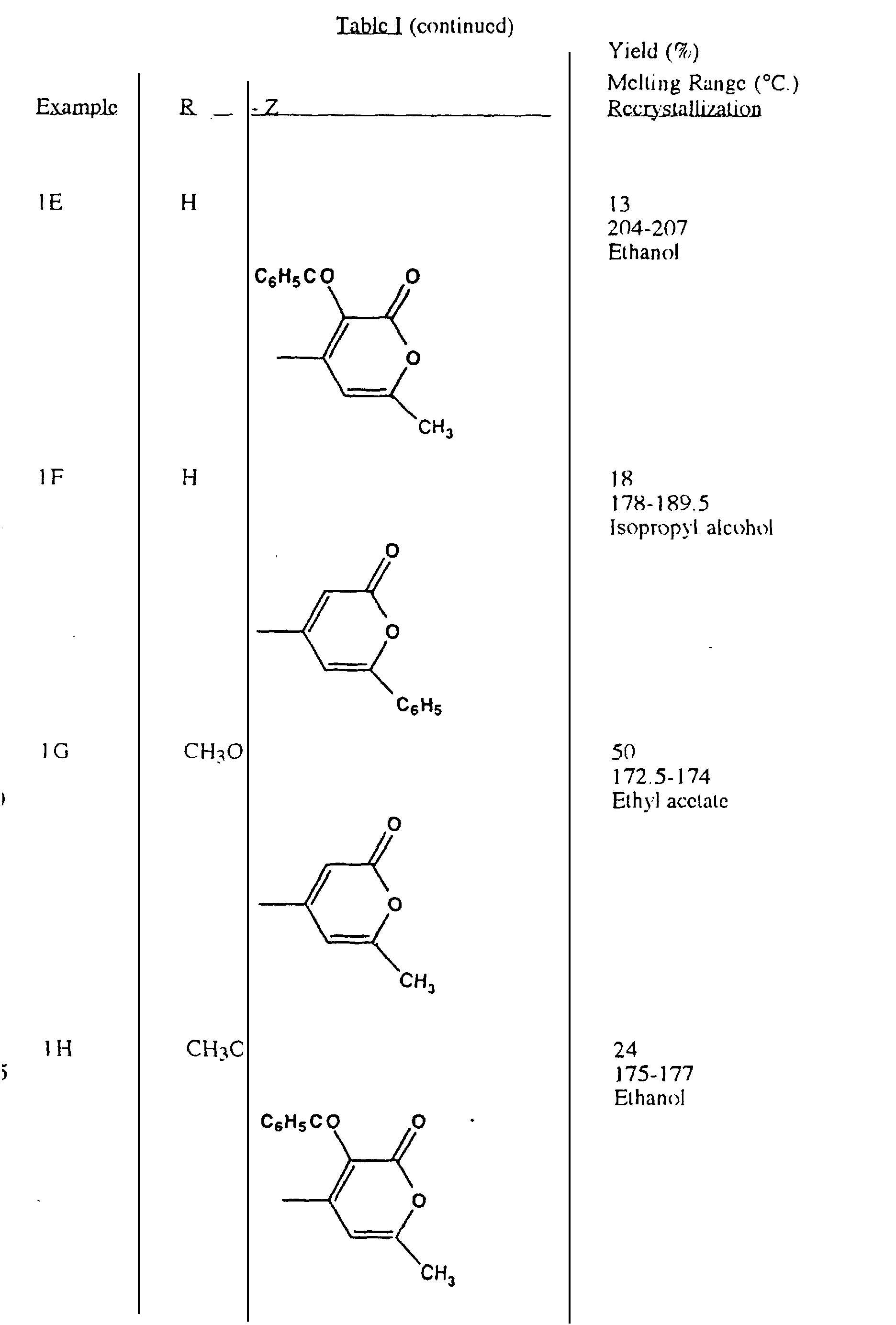

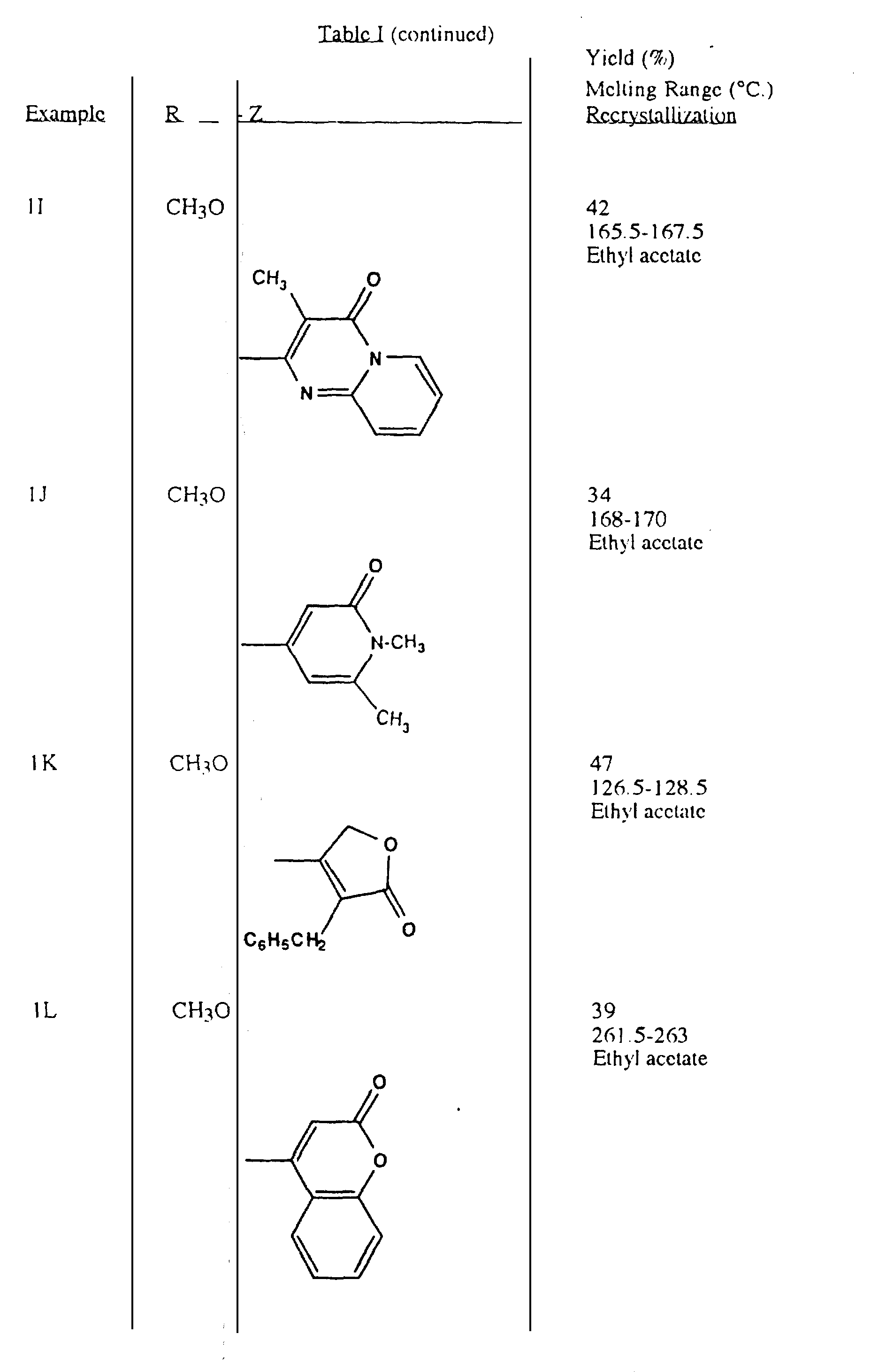

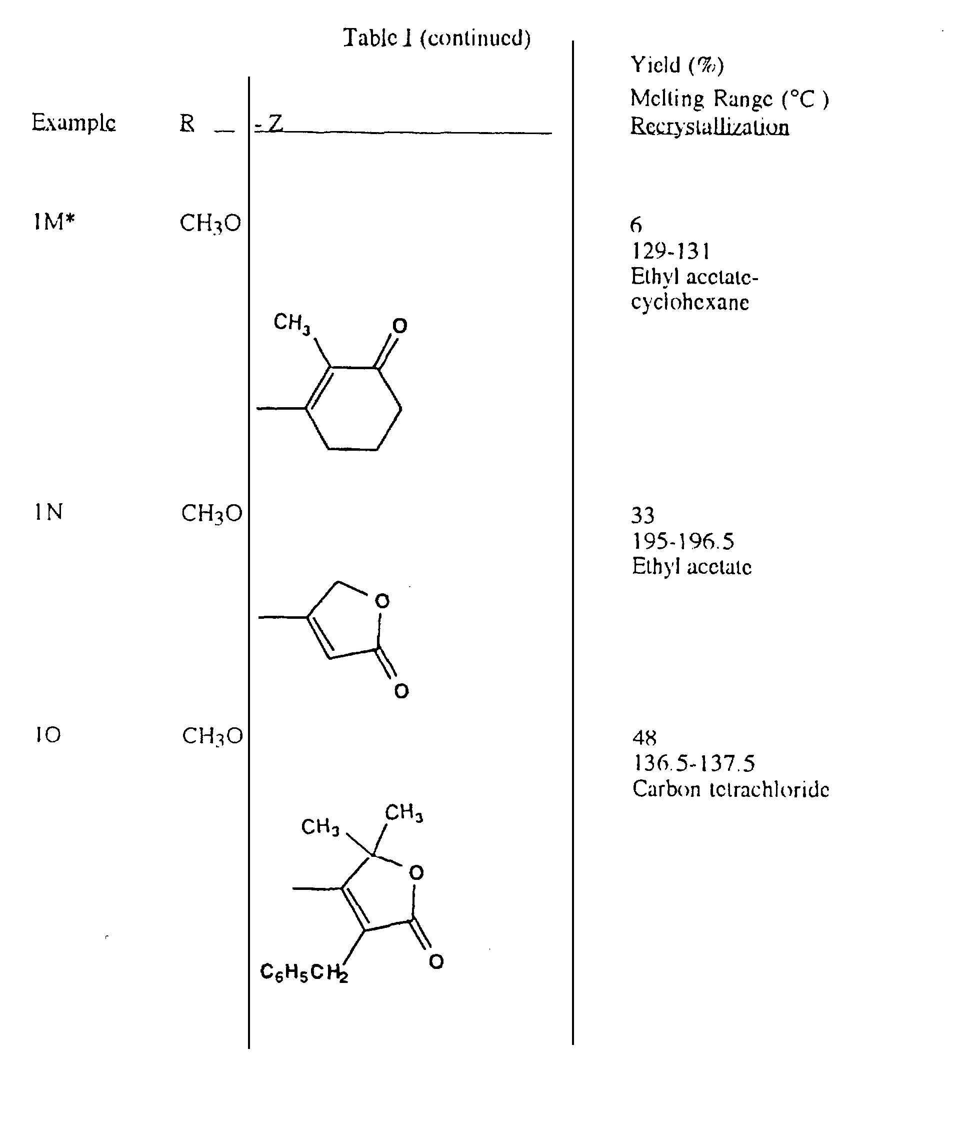

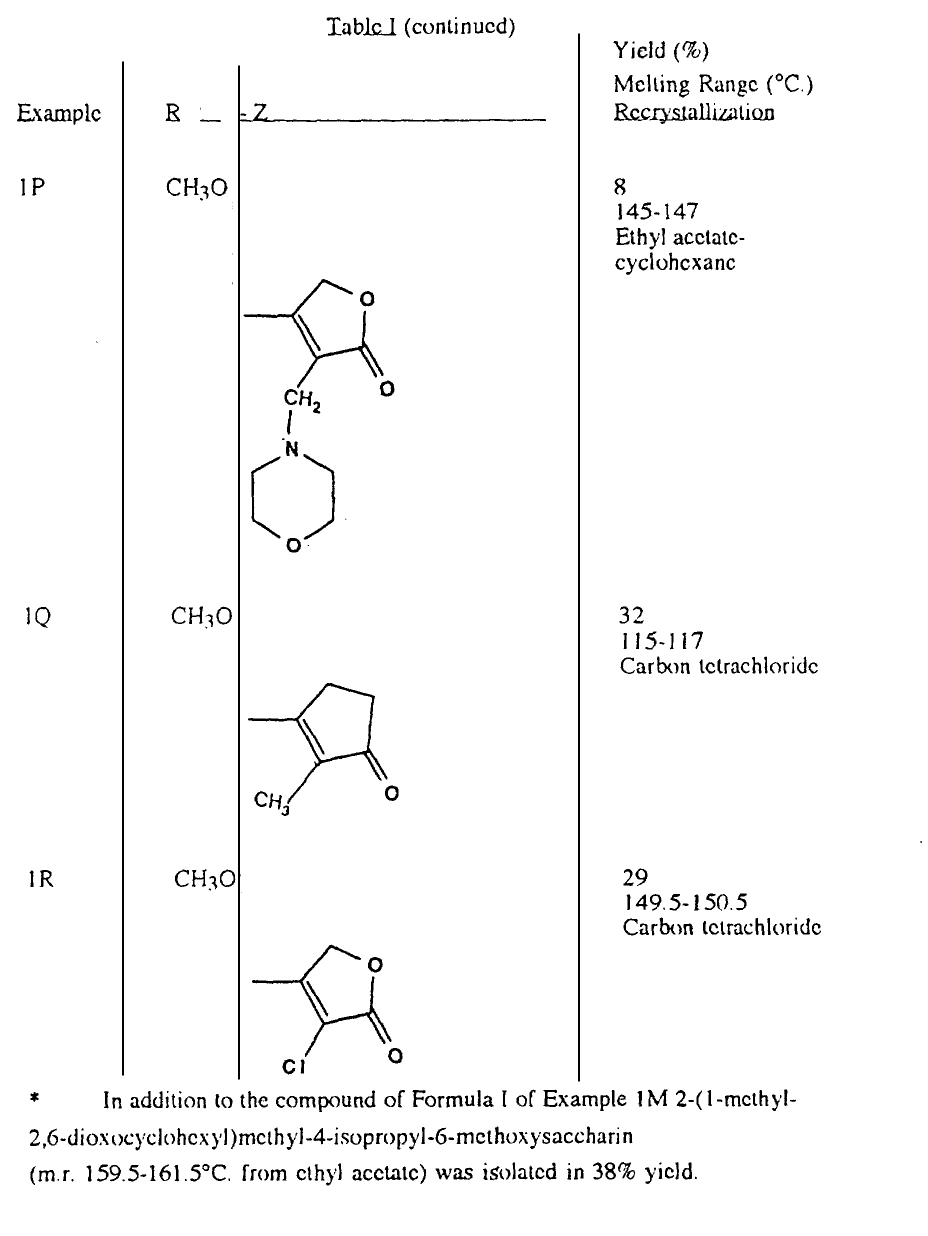

### Example 2

### 2-(1-methylcarbostyryl-4-yl)oxymethyl-4-isopropyl-6-methoxysaccharin

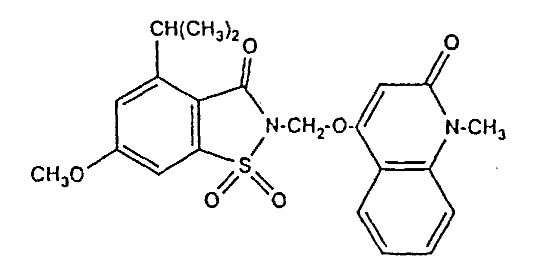

4-Hydroxy-1-methylcarbostyryl (1.44 g) was added with stirring to a suspension of sodium hydride (60% dispersion in mineral oil, 0.36 g) in dimethylformamide. The mixture was heated at 100°C. with continued stirring for three-quarters of an hour, then sonicated at room temperature for half an hour. 2-Chloromethyl-4-isopropyl-6-methoxysaccharin (1.83 g) was added. The resulting mixture was heated at 100°C. with continued stirring for two hours, then poured into water (300 ml). Hydrochloric acid (1N, 25 ml) was added, and the mixture was extracted with chloroform. The chloroform extract was dried over sodium sulfate and stripped of chloroform. Flash chromatography of the resulting solid (3.3 g) on silica gel using ethyl acetate-hexane (1:1) as eluant and recrystallization of the resulting solid (0.7 g, 28% yield) from ethanol (90%) gave the title compound as a yellow solid (0.39 g, 15% yield, m.r. 198-200°C.).

### Example 3A

### 2-(1-phenyl-3-acetylcarbostyryl-4-yl)oxymethyl-4-isopropyl-6-methoxysaccharin

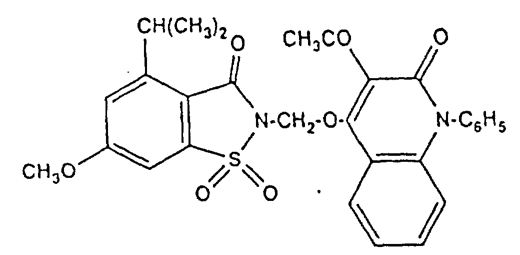

A mixture of 3-acetyl-4-hydroxy-1-phenylcarbostyril (0.52 g), potassium carbonate (0.28 g) and dimethylformamide (7 ml) was stirred at room temperature for half an hour. 2-Chloromethyl-4-isopropyl-6-methoxysaccharin (0.71 g) was added and the mixture was sonicated for one hour at 40°C. and stirred at room temperature for 16 hours. Further sonication did not change the extent of reaction as shown by thin layer chromatography. The reaction mixture was poured into water and the mixture was extracted with ethyl acetate. The ethyl acetate extract was dried and stripped of ethyl acetate. Medium pressure liquid chromatography of the residue (0.5 g) on silica gel using ethyl acelate-hexane (3:7) as eluant and recrystallization of the product from methanol gave the title compound (0.24 g, 24% yield, m.r. 210-212°C.).

### Example 3B

### 2-(1-methyl-3-acetylcarbostyryl-4-yl)oxymethyl-4-isopropyl-6-methoxysaccharin

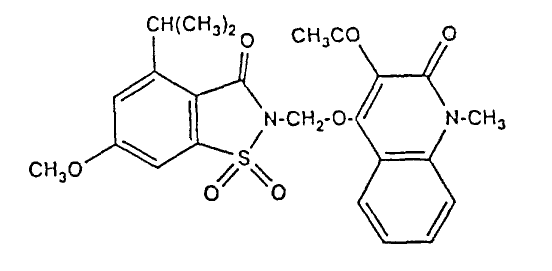

By a method similar to that of Example 3A above 3-acetyl-4-hydroxy-1-methylcarbostyryl (0.63 g) was condensed with 2-chloromethyl-4-isopropyl-6-methoxysaccharin (1.10 g) and the product was purified by crystallization from methanol-ether to give the title compound as an off-white powder (0.28 g, 20% yield, m.r. 194-196°C.).

### Example 4A

### 2-(2,5-dihydro-2-oxo-3-phenylfuran-4-yl)oxymethyl-4-isopropyl-6-methoxysaccharin

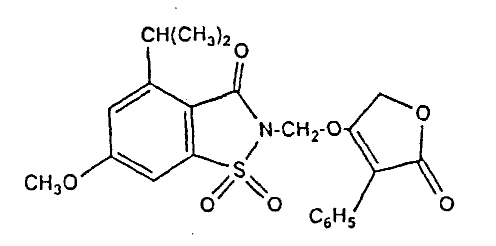

Cesium carbonate (0.82 g) was added to a solution of 3-phenyl-2(5H)-furanone (0.88 g) in methanol (15 ml) and the mixture was stirred at room temperature for one hour and stripped of methanol. 2-Chloromethyl-4-isopropyl-6-methoxysaccharin (1.52 g) was added to a solution of the residue in dimethylformamide (15 ml) and the mixture was stirred at room temperature for two days. Silica gel (4.4 g) was added, volatiles were removed under vacuum, and the solid was subjected to flash chromatography on silica gel using ethyl acetate-hexane (3:7) as eluant. Recrystallization of the product from ethanol-ether gave the title compound as a colorless solid (0.45 g, 20% yield, m.r. 180-182°C.).

### Examples 4B-4G

By methods similar to that of Example 4A above the compounds identified in Table II below were prepared from 2-chloromethyl-4-isopropyl-6-methoxysaccharin and the corresponding compound of Formula III (H-O-Z).

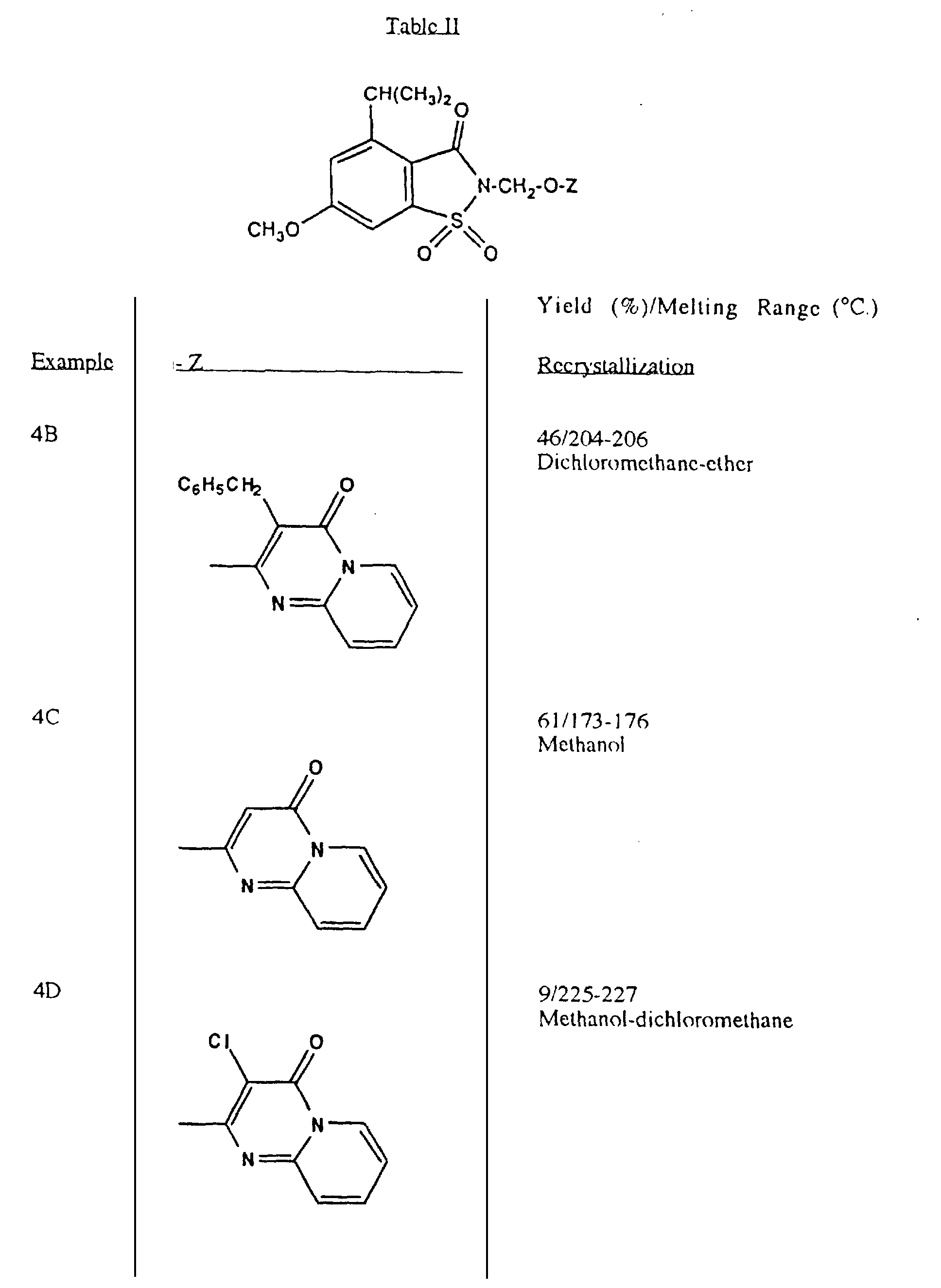

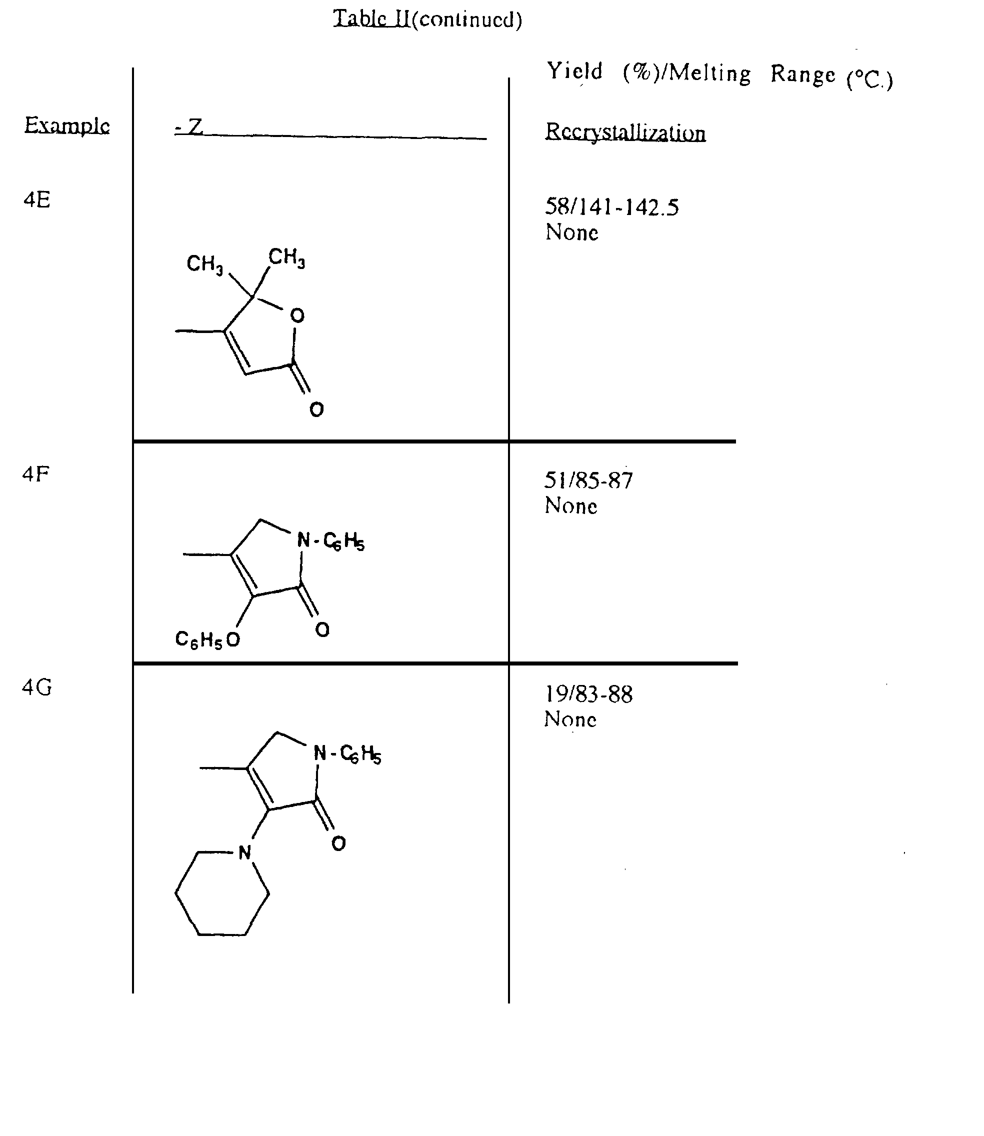

### Example 5

### 2-[3-(4-morpholinymethyl)-4-oxo-4H-pyrido]1.2-a]pyrimidin-2-yl]oxymethyl-4-isopropyl-6-methoxysaccharin

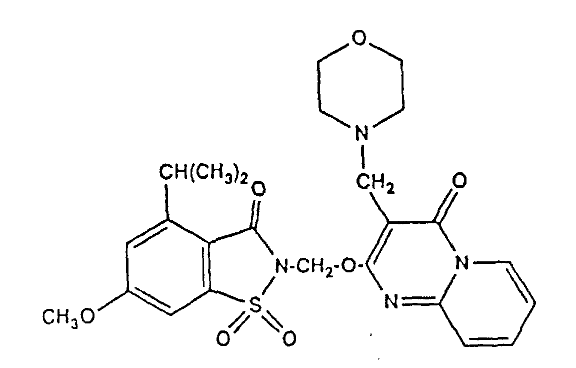

A mixture of 3-(4-morpholinylmethyl)pyrido[1,2-a]pyrimidine-2,4-dione hydrochloride (0.6 g) and potassium t-butoxidc (0.49 g) in dimethyl-formamide (20 ml) was stirred at room temperature for five minutes. 2-Chloromethyl-4-isopropyl-6-methoxysaccharin (0.61 g) was added and stirring was continued at room temperature for one hour. The reaction mixture was diluted with water (50 ml) and extracted with dichloromethane (3 x 200 ml). The dichloromethane extract was dried over sodium sulfate and stripped of volatiles, finally under high vacuum. The crude product (780 mg) was combined with that (0.65 g) from another run at the same scale using sodium hydride (60% dispersion in mineral oil, 0.19 g) instead of potassium t-butoxide and purified by column chromatography on silica gel with dichloromethane-methanol (95:5) as eluant affording the title compound (500 mg, 23% yield), part of which was recrystallized from ethanol (m.r. 177-180°C.).

As stated above the compounds of Formula I inhibit the enzymatic activity of proteolytic enzymes and are useful in the treatment of degenerative diseases. More particularly they inhibit human leukocyte elastase and chymotrypsin-like enzymes and are useful in the treatment of emphysema, rheumatoid arthritis, pancreatitis, cystic fibrosis, chronic bronchitis, adult respiratory distress syndrome, inflammatory bowel disease, psoriasis, bullous pemphigoid and alpha-1-antitrypsin deficiency. This utility was demonstrated by an in vitro test of the inhibition of the compounds of Formula I against human leukocyte elastase.

Measurement of the inhibition constant (Kᵢ) of a human leukocyte elastase inhibitor complex has been described (Cha, Biochemical Pharmacology, vol 24, pp. 2177-2185, 1975) for truly reversible inhibition constants usually concerning competitive inhibitors. The compounds of Formula I do not form truly reversible inhibitor complexes but rather are consumed by the enzyme to some extent. Kᵢ^{*}, which is defined as the rate of reactivation of the enzyme divided by the rate of inactivation of the enzyme (k_{off}/kₒₙ), was therefore determined instead. The values of k_{off} and kₒₙ were measured and Kᵢ^{*} was then calculated.

The value of kₒₙ was determined by measuring the enzyme activity of an aliquot of the enzyme as a function of the time after addition of the test compound (inhibitor). By plotting the log of the enzyme activity against time an observed rate of inactivation (k_{obs}) was obtained by the equation k_{obs} = In 2/t_{1/2} wherein t_{1/2} is the time required for the enzyme activity to decrease by 50%. The value of kₒₙ was then obtained by the equation kₒₙ = k_{obs}/[I] wherein [I] is the concentration of the inhibitor. The value of k_{off} was similarly determined, and Kᵢ^{*} was then obtained by the equation Kᵢ^{*} = k_{off}/kₒₙ.

The results shown in Table III were obtained for compounds of Formula I of the examples.

**Table III**

| **Inhibition of Human Leukocyte Elastase** | |
|---|---|
| Compound of Example | Kᵢ^{*}(nM) |
| 1A | 0.4 |
| 1B | 0.22 |
| 1C | 0.7 |
| 1D | 0.25 |
| 1E | 0.035 |
| 1F | 0.22 |
| 1G | 0.05 |
| 1H | 0.027 |
| 1I | 0.25 |
| 1J | 0.85 |
| 1K | 0.013 |
| 1L | 0.10 |
| | 0.09 |
| 1M | 0.18 |
| 1N | 0.052 |
| 1O | 0.016 |
| 1P | 0.041 |
| 1Q | 0.06 |
| 1R | 0.038 |
| 2 | 0.5 |
| 3A | 0.43 |
| 3B | 0.27 |
| 4A | 0.025 |
| 4B | 0.9 |
| 4C | 0.078 |
| 4D | 0.066 |
| 4E | 0.093 |
| 4F | 0.06 |
| 4G | 0.2 |
| 5 | 0.23 |

The proteolytic enzyme inhibiting amount of the compound of Formulae I can be estimated from the results of the test for human leukocyte elastase inhibition and can additionally be varied for a particular patient depending on the physical condition of the patient, the route of administration, the duration of treatment and the response of the patient. An effective dose of the compound of Formula I can thus only be determined by the clinician after consideration of all pertinent criteria and exercise of best judgment on behalf of the patient.
A compound of Formulae I can be prepared for pharmaceutical use by incorporating it in a pharmaceutical composition for oral, parenteral or aerosol inhalation administration, which can be in solid or liquid dosage form including tablets, capsules, solutions, suspensions and emulsions and which can include one or more suitable adjuvants. Solid unit dosages in the form of tablets or capsules for oral administration are preferred. For this purpose the adjuvant can be for example one or more of calcium carbonate, starch, lactose, talc, magnesium stearate and gum acacia. The compositions are prepared by conventional pharmaceutical techniques.

## Claims

1. A compound having the structural formula (I): 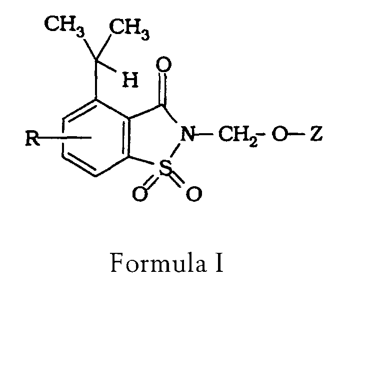 wherein
R is hydrogen or methoxy; and
Z is selected from the following radicals : 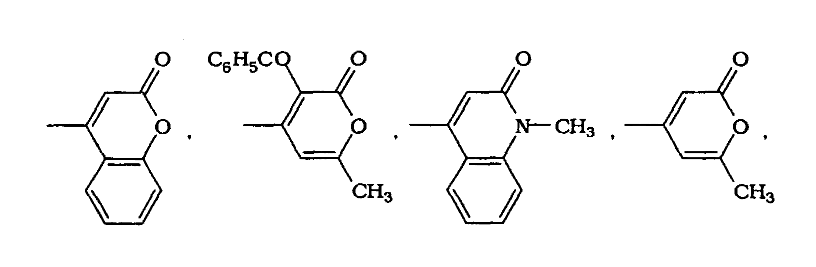 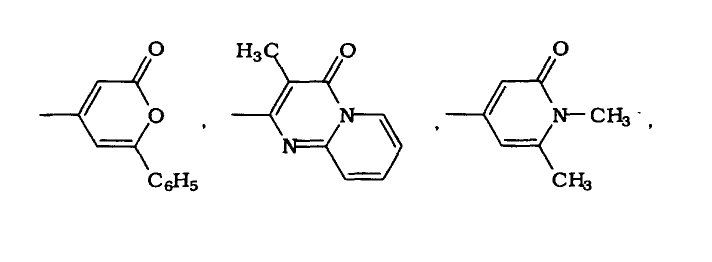 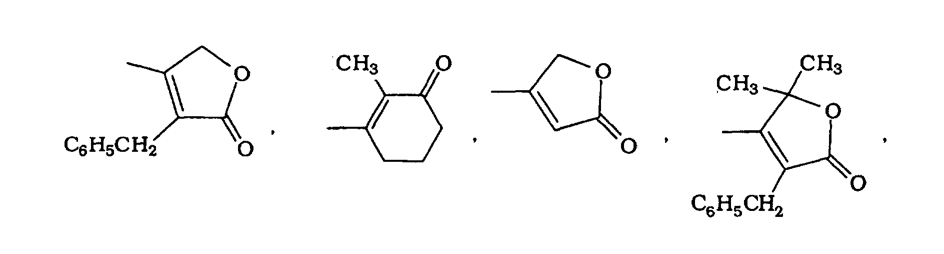 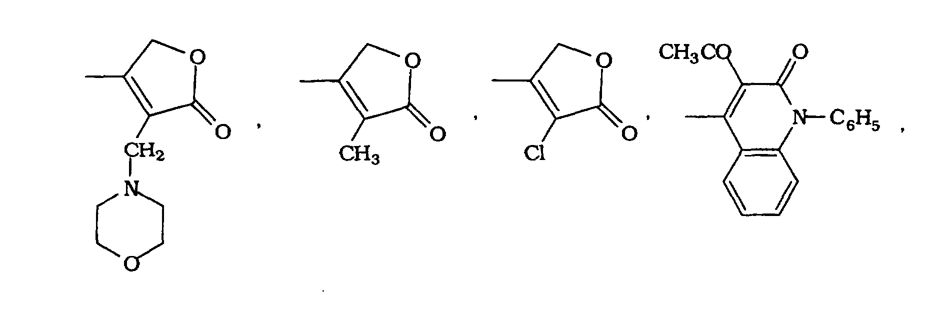 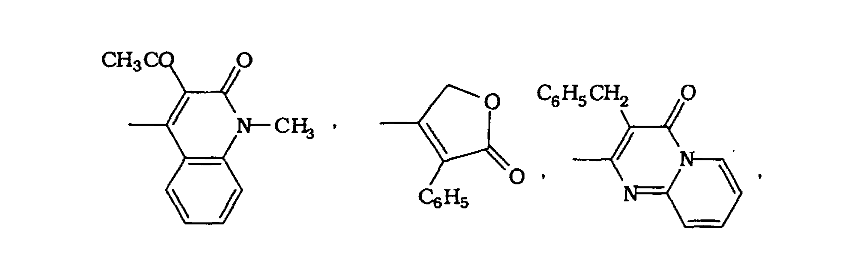 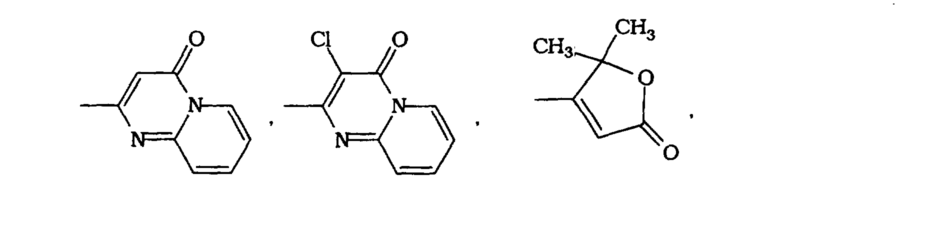 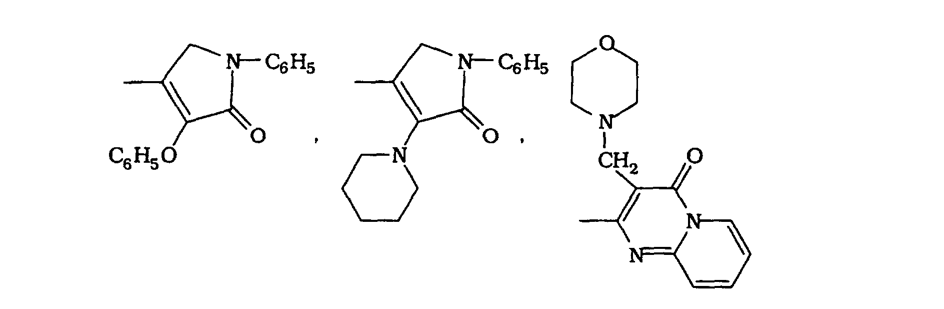 or a pharmaceutically acceptable acid addition salt thereof if the compound has a basic functional group or a pharmaceutically acceptable base addition salt thereof if the compound has an acidic functional group.

2. A compound according to claim 1 wherein R is hydrogen or 6-methoxy.

3. A process for preparing a compound of Formula I as claimed in claim 1 which comprises condensing the corresponding compound having the structural formula (II): 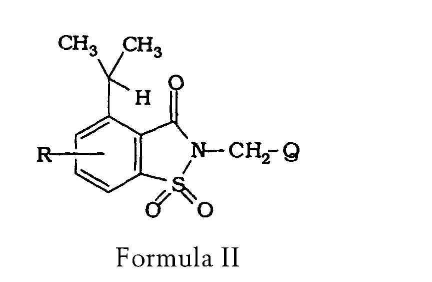 in which R is as defined in claim 1,
wherein Q is chloro or bromo with the corresponding compound having the structural formula (III):
H-O-Z Formula III
wherein Z is as defined in claim 1, in the presence of a base or with a corresponding basic salt of the compound of Formula III.

4. A pharmaceutical composition comprising a proteolytic enzyme inhibiting concentration of a compound as claimed in any one of claims 1 and 2 and a pharmaceutical carrier.

5. The use of a compound as claimed in any one of claims 1 and 2 for the preparation of a medicament for the treatment of a degenerative disease in a patient in need of such treatment.

## Patentansprüche

1. Verbindung der Strukturformel (I): 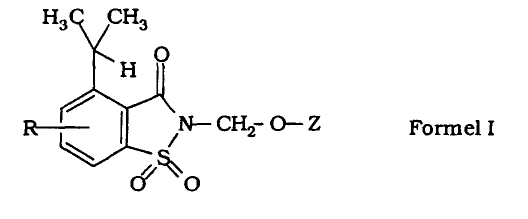 in der
R₁ Wasser oder Methoxy bedeutet; und
Z aus den folgenden Resten ausgewählt ist: 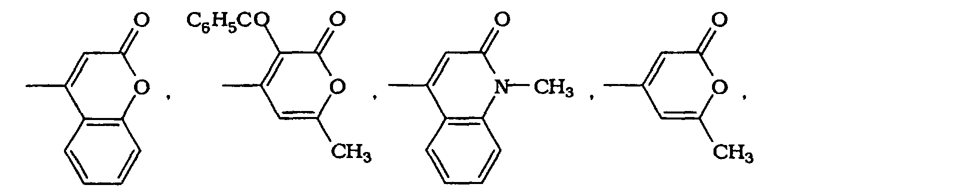 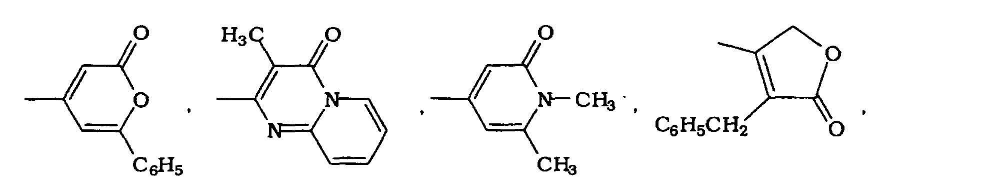 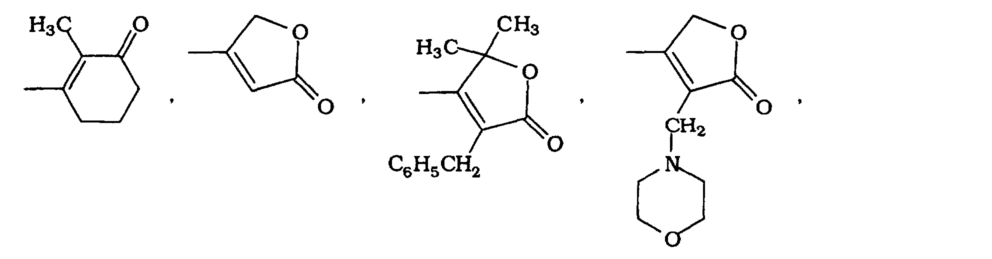 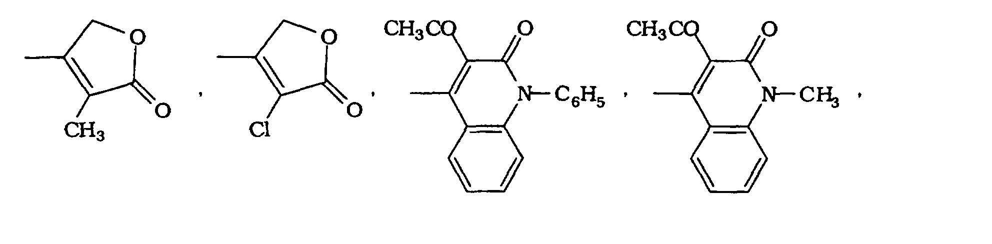 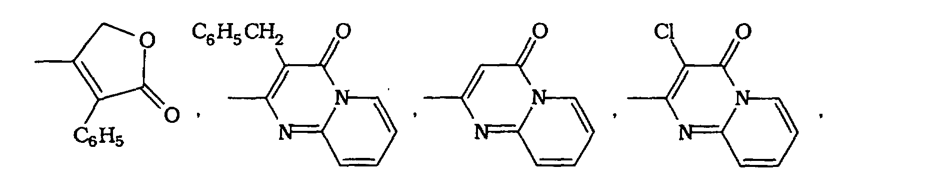 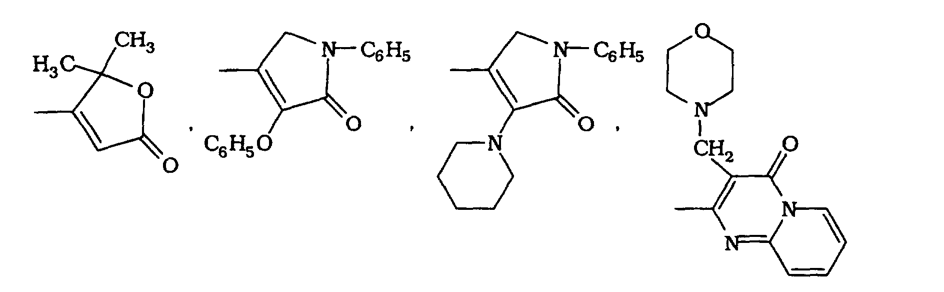 oder ein pharmazeutisch annehmbares Säureadditionssalz davon, wenn die Verbindung eine basische funktionelle Gruppe aufweist, oder ein pharmazeutisch annehmbares Basenadditionssalz davon, wenn die Verbindung eine saure funktionelle Gruppe aufweist.

2. Verbindung nach Anspruch 1, worin R Wasserstoff oder 6-Methoxy bedeutet.

3. Verfahren zur Herstellung einer Verbindung der Formel I, wie sie in Anspruch 1 beansprucht ist, welches darin besteht, die entsprechende Verbindung mit der Strukturformel (II): 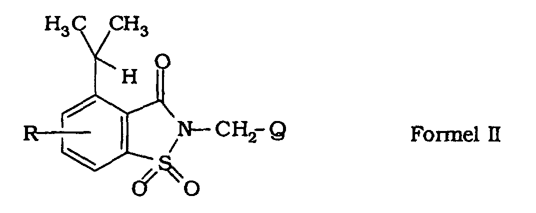 in der R die in Anspruch 1 angegebenen Bedeutungen besitzt,
und Q Chlor oder Brom bedeutet, mit der entsprechenden Verbindung der Strukturformel (III):
H - O - Z Formel III
in der Z die in Anspruch 1 angegebenen Bedeutungen besitzt. in Gegenwart einer Base oder mit einem entsprechenden Basensalz der Verbindungen der Formel III zu kondensieren.

4. Pharmazeutische Zubereitung, umfassend eine in irgendeinem der Ansprüche 1 und 2 beanspruchte Verbindung in einer proteolytische Enzyme inhibierenden Konzentration und ein pharmazeutisches Trägermaterial.

5. Verwendung einer Verbindung, wie sie in einem der Ansprüche 1 oder 2 beansprucht ist, für die Herstellung eines Arzneimittels zur Behandlung von degenerativen Erkrankungen bei Patienten, die einer solchen Behandlung bedürfen.

## Revendications

1. Composé de formule développée (I) 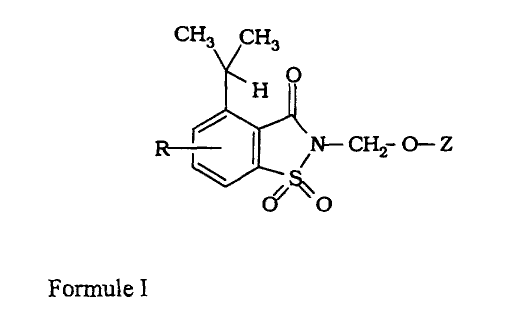 dans laquelle
R est un hydrogène ou un méthoxy; et
Z est choisi parmi les radicaux suivants : 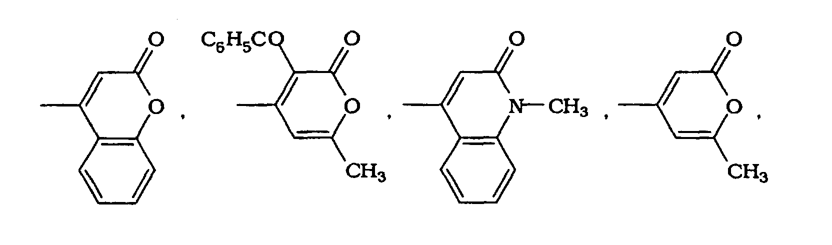 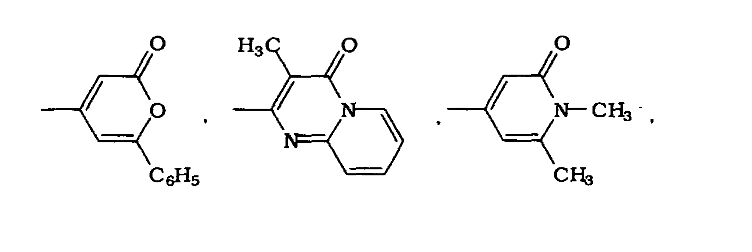 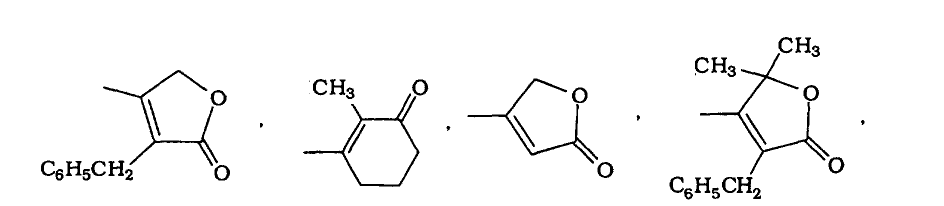 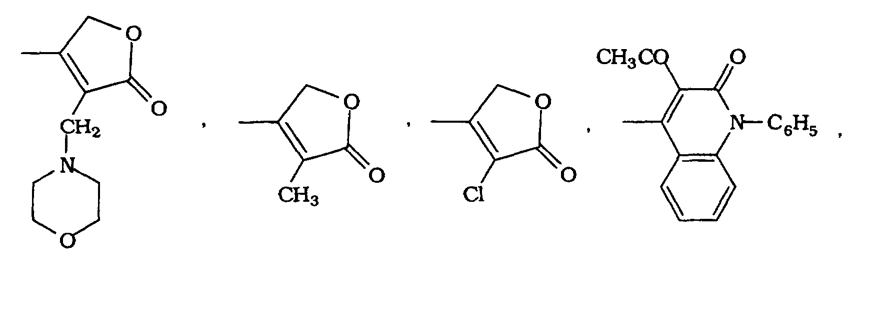 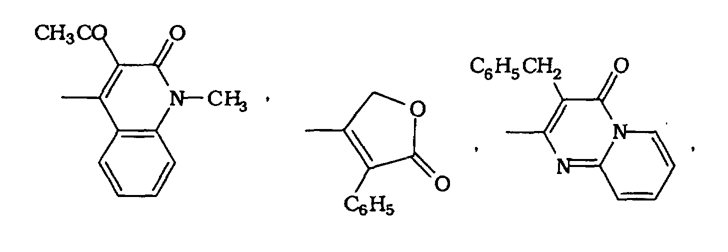 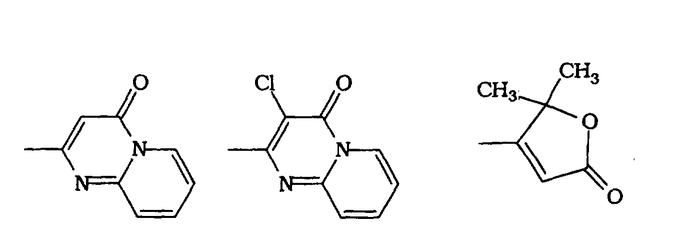 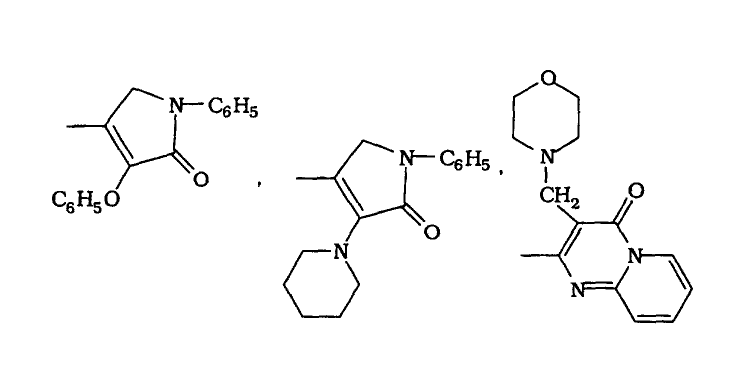 ou un sel d'addition acide pharmaceutiquement acceptable de ce composé si ce composé a un groupe fonctionnel basique ou un sel d'addition basique pharmaceutiquement acceptable de ce composé si ce composé a un groupe fonctionnel acide.

2. Composé selon la revendication 1 dans lequel R est un hydrogène ou un 6-méthoxy.

3. Procédé de préparation d'un composé de formule I comme revendiqué dans la revendication 1 qui comprend la condensation du composé correspondant de formule développée (II) 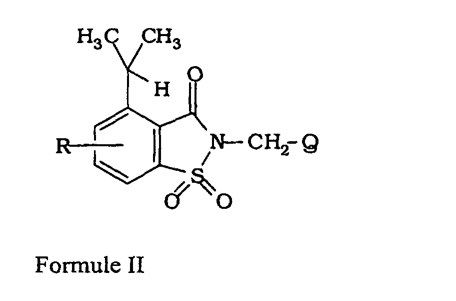 dans laquelle R est comme défini dans la revendication 1,
dans laquelle Q est un chloro ou un bromo, le composé correspondant ayant la formule développée (III):
H-O-Z Formule III
dans laquelle Z est comme défini dans la revendication 1, en présence d'une base ou avec un sel basique correspondant du composé de formule III.

4. Composition pharmaceutique comprenant une concentration d'un composé comme revendiqué dans l'une quelconque des revendications 1 et 2 inhibant les enzymes protéolytiques et un porteur pharmaceutique.

5. Utilisation d'un composé comme revendiqué dans l'une quelconque des revendications 1 et 2 pour la préparation d'un médicament pour le traitement d'une maladie dégénérescente chez un patient ayant besoin d'un tel traitement.
